# EUROPEAN PATENT APPLICATION

(11) **EP 4 751 588 A1**
(43) Date of publication of application: **03.06.2026**
(21) Application number: 24844813.6
(22) Date of filing: 24.07.2024
(51) Int. Cl.: A24F 40/40, A24F 40/10, A24F 40/42, A24F 40/46, A24F 40/48, A24F 40/44, A61M 11/00, B05B 17/06

(54) **ATOMIZER, ULTRASONIC ATOMIZATION SHEET ASSEMBLY, AND AEROSOL GENERATION APPARATUS**

(30) Priority: 25.07.2023 CN 202310920082; 25.07.2023 CN 202310922055
(71) Applicant: Smoore International Holdings Limited, Grand Cayman, KY1-1111 (KY)
(72) Inventor: LIAO, Peilong, Shenzhen, Guangdong 518102 (CN); FAN, Pengyuan, Shenzhen, Guangdong 518102 (CN); WAN, Wenlong, Shenzhen, Guangdong 518102 (CN); XIE, Yajun, Shenzhen, Guangdong 518102 (CN); DENG, Jingjing, Shenzhen, Guangdong 518102 (CN); JIANG, Taoming, Shenzhen, Guangdong 518102 (CN); ZHANG, Hao, Shenzhen, Guangdong 518102 (CN); LIN, Ping, Shenzhen, Guangdong 518102 (CN); XIE, Rui, Shenzhen, Guangdong 518102 (CN)
(74) Representative: Winter, Brandl - Partnerschaft mbB
(86) International application number: PCT/CN2024/107352
(87) International publication number: WO 2025/021122

(57) **Abstract**

This application relates to an atomizer and an aerosol generating device. The atomizer includes a housing assembly, where an air outlet channel is formed in the housing assembly; an atomization base, where at least a part of the atomization base is disposed in the housing assembly; and an atomization core disposed on the atomization base, where the atomization core includes an ultrasonic atomization chip assembly, the ultrasonic atomization chip assembly is used for atomizing an aerosol generating substrate, the ultrasonic atomization chip assembly has an aerosol outlet area used for discharging an aerosol, and the aerosol outlet area faces the top side of the atomizer. The bottom end of the air outlet channel covers the periphery of the aerosol outlet area, to cause the aerosol generated through atomization to be discharged from the housing assembly through the air outlet channel. According to embodiments of the present disclosure, the bottom end of the air outlet channel of the atomizer covers the periphery of the aerosol outlet area, used for discharging the aerosol, of the ultrasonic atomization chip assembly. In this way, the aerosol generated through atomization can be directly discharged from the housing assembly through the air outlet channel for user inhalation, which shortens a flow path of the aerosol, and reduces loss of the aerosol.

## Description

### CROSS-REFERENCE TO RELATED APPLICATIONS

This application is based on and claims priority to Chinese Patent Application No. 202310920082.0, filed on July 25, 2023, and Chinese Patent Application No. 202310922055.7, filed on July 25, 2023, which is incorporated herein by reference in its entirety.

### TECHNICAL FIELD

This application relates to the technical field of electronic atomization, and in particular, to an atomizer, an ultrasonic atomization chip assembly, and an aerosol generating device.

### BACKGROUND

Typically, an aerosol generating device includes an atomizer and a power supply component electrically connected to the atomizer. The atomizer is capable of, under the electrical drive of the power supply component, atomizing an aerosol generating substrate stored in a liquid storage cavity, to generate an aerosol for user inhalation.

In the related technology, during discharge, an aerosol generated through atomization may experience significant loss, which adversely affects user experience.

### SUMMARY

In view of this, embodiments of the present disclosure provide an atomizer, an ultrasonic atomization chip assembly, and an aerosol generating device, to reduce loss of an aerosol generated through atomization, thereby improving user experience.

An embodiment of the present disclosure provides an atomizer. The atomizer includes:
a housing assembly, where an air outlet channel is formed in the housing assembly;
an atomization base, where at least a part of the atomization base is disposed in the housing assembly; and
an atomization core, disposed on the atomization base and including an ultrasonic atomization chip assembly, where the ultrasonic atomization chip assembly is used for atomizing an aerosol generating substrate, the ultrasonic atomization chip assembly has an aerosol outlet area used for discharging an aerosol, and the aerosol outlet area faces the top side of the atomizer, where
the bottom end of the air outlet channel covers the periphery of the aerosol outlet area, to cause the aerosol generated through atomization to be discharged from the housing assembly through the air outlet channel.

In some embodiments, the atomization base is formed with an air inlet channel, the side wall at the bottom end of the air outlet channel is formed with at least one notch, and the air inlet channel communicates with the air outlet channel through the notch.

In some embodiments, the air outlet channel linearly extends in a top-bottom direction of the atomizer.

In some embodiments, the length of the air outlet channel is not greater than 35 mm.

In some embodiments, the length of the air outlet channel is between 15 mm and 35 mm; and/or the equivalent diameter of the air outlet channel is between 2.5 mm and 5.0 mm.

In some embodiments, a liquid storage cavity is formed in the housing assembly, the liquid storage cavity is located on the top side of the atomization base, the air outlet channel is located in the middle area of the liquid storage cavity, and the ultrasonic atomization chip assembly is arranged perpendicular to the top-bottom direction of the atomizer.

In some embodiments, the atomization base includes: an atomization top base, an atomization bottom base, and a first sealing member. The atomization bottom base is located on the bottom side of the atomization top base, and the first sealing member is located on the top side of the atomization top base.

In some embodiments, the bottom surface of the first sealing member is formed with a plurality of convex points;
the atomization top base is formed with a groove, and the ultrasonic atomization chip assembly is disposed in the groove; and
the first sealing member abuts against the ultrasonic atomization chip assembly through the convex points, to fix the ultrasonic atomization chip assembly in the groove.

In some embodiments, a spacing area is formed between the bottom surface of the first sealing member and the top surface of the ultrasonic atomization chip assembly, and the convex points are located in the spacing area; and
the atomization top base is formed with at least one communication groove, the atomization bottom base is formed with at least one air inlet hole, the communication groove communicates with the air inlet hole and the spacing area, and the air inlet hole, the communication groove, and the spacing area define at least a part of the air inlet channel.

In some embodiments, the atomizer includes: a second sealing member. The second sealing member is formed with a first through hole, an annular groove is formed in the hole wall of the first through hole, and the second sealing member surrounds the ultrasonic atomization chip assembly, and the edge of the ultrasonic atomization chip assembly is embedded into the annular groove.

In some embodiments, the ultrasonic atomization chip assembly includes: a piezoelectric ceramic sheet and a microporous sheet, where
the microporous sheet is superposed on the bottom side of the piezoelectric ceramic sheet and is connected to the piezoelectric ceramic sheet, a second through hole is formed in the middle area of the piezoelectric ceramic sheet, and micropores are formed in an area, corresponding to the second through hole, of the microporous sheet.

In some embodiments, the micropores include first micropores. The first micropores form a first aerosol outlet on the surface on the side, facing the piezoelectric ceramic sheet, of the microporous sheet, and the pore size of the first aerosol outlet is not greater than 2.0 µm.

In some embodiments, the pore size of the first aerosol outlet is between 1.1 µm and 2.0 µm.

In some embodiments, the first micropores form a first liquid inlet on the surface on the side, facing away from the piezoelectric ceramic sheet, of the microporous sheet, and the pore size of the first liquid inlet is between 20 µm and 50 µm.

In some embodiments, a number of micropores is between 500 and 4,000; and/or a spacing distance between any two adjacent micropores is between 40 µm and 100 µm.

In some embodiments, the thickness of the piezoelectric ceramic sheet is between 0.4 mm and 0.8 mm; and/or the diameter of the piezoelectric ceramic sheet is between 12 mm and 14 mm.

In some embodiments, the thickness of the microporous sheet is between 0.2 mm and 1.0 mm; and/or the diameter of the microporous sheet is between 12 mm and 14 mm.

In some embodiments, the atomization base is formed with a liquid inlet channel, the side, facing the liquid inlet channel, of the central area of the microporous sheet protrudes to form a protrusion, and the micropores are formed in the protrusion.

In some embodiments, the atomization core further includes: a liquid guiding member. The liquid guiding member is located in the liquid inlet channel, abuts against the protrusion, and is used for guiding the aerosol generating substrate to the ultrasonic atomization chip assembly.

In some embodiments, a material of the piezoelectric ceramic sheet is one of lead zirconate titanate, barium carbonate, and potassium sodium niobate; and/or a material of the microporous sheet is one of stainless steel, nickel-cobalt alloy, titanium alloy, copper alloy, and palladium-nickel alloy.

In some embodiments, the micropores further include second micropores. The second micropores form a second aerosol outlet on the surface on the side, facing the piezoelectric ceramic sheet, of the microporous sheet, and the pore size of the second aerosol outlet is between 5 µm and 20 µm.

In some embodiments, a ratio of a number of first micropores to a number of second micropores ranges from 7: 1 to 10: 1.

An embodiment of the present disclosure further provides an ultrasonic atomization chip assembly used in the atomizer according to any one of the foregoing embodiments. The ultrasonic atomization chip assembly includes:
a piezoelectric ceramic sheet, where a second through hole is formed in the middle area of the piezoelectric ceramic sheet; and
a microporous sheet, coaxially superposed on the first axial side of the piezoelectric ceramic sheet and connected to the piezoelectric ceramic sheet, where micropores are formed in an area, corresponding to the second through hole, of the microporous sheet, where
the micropores include first micropores, the first micropores form a first aerosol outlet on the surface on the side, facing the piezoelectric ceramic sheet, of the microporous sheet, and the pore size of the first aerosol outlet is not greater than 2.0 µm.

In some embodiments, the pore size of the first aerosol outlet is between 1.1 µm and 2.0 µm.

In some embodiments, the first micropores form a first liquid inlet on the surface on the side, facing away from the piezoelectric ceramic sheet, of the microporous sheet, and the pore size of the first liquid inlet is between 20 µm and 50 µm.

In some embodiments, a number of micropores is between 500 and 4,000; and/or a spacing distance between any two adjacent micropores is between 40 µm and 100 µm.

In some embodiments, the thickness of the piezoelectric ceramic sheet is between 0.4 mm and 0.8 mm; and/or the diameter of the piezoelectric ceramic sheet is between 12 mm and 14 mm.

In some embodiments, the thickness of the microporous sheet is between 0.2 mm and 1.0 mm; and/or the diameter of the microporous sheet is between 12 mm and 14 mm.

In some embodiments, the side, facing away from the piezoelectric ceramic sheet, of the central area of the microporous sheet protrudes to form a protrusion, and the micropores are located in the protrusion.

In some embodiments, a material of the piezoelectric ceramic sheet is one of lead zirconate titanate, barium carbonate, and potassium sodium niobate; and/or a material of the microporous sheet is one of stainless steel, nickel-cobalt alloy, titanium alloy, copper alloy, and palladium-nickel alloy.

In some embodiments, the micropores further include second micropores. The second micropores form a second aerosol outlet on the surface on the side, facing the piezoelectric ceramic sheet, of the microporous sheet, and the pore size of the second aerosol outlet is between 5 µm and 20 µm.

In some embodiments, a ratio of a number of first micropores to a number of second micropores ranges from 7: 1 to 10: 1.

An embodiment of the present disclosure further provides an aerosol generating device. The aerosol generating device includes:
a power supply component; and
the atomizer according to any one of the foregoing embodiments, where
the power supply component is electrically connected to the atomizer and is used for supplying power to the atomizer.

The technical solutions provided in the embodiments of the present disclosure may include the following beneficial effects:
The atomizer provided in the embodiments of the present disclosure includes the housing assembly, the atomization base, and an atomization core. The air outlet channel is formed in the housing assembly. The atomization core includes the ultrasonic atomization chip assembly. The ultrasonic atomization chip assembly is used for atomizing the aerosol generating substrate. In addition, the ultrasonic atomization chip assembly has the aerosol outlet area used for discharging an aerosol generated through atomization. The bottom end of the air outlet channel covers the periphery of the aerosol outlet area. In this way, the aerosol generated through atomization can be directly discharged from the housing assembly through the air outlet channel for user inhalation, thereby shortening a flow path of the aerosol, reducing a possibility of condensation of the aerosol, reducing loss of the aerosol, and improving user experience.

### BRIEF DESCRIPTION OF THE DRAWINGS

FIG. 1 is a schematic structural diagram of an aerosol generating device according to an exemplary embodiment;
FIG. 2 is a schematic diagram of an exploded structure of an atomizer of the aerosol generating device shown in FIG. 1;
FIG. 3 is a schematic sectional view of an atomizer shown in FIG. 2;
FIG. 4 is a schematic diagram of exploded structures of an atomization top base, an atomization bottom base, and an atomization core of the atomizer shown in FIG. 2;
FIG. 5 is a schematic sectional view of fit between an atomization base and an atomization core of the atomizer shown in FIG. 2, where an arrow schematically indicates a flow direction of an aerosol generating substrate;
FIG. 6 is a sectional view of fit between an atomization base and an atomization core of the atomizer shown in FIG. 2 from another perspective, where an arrow schematically indicates a flow direction of external air;
FIG. 7 is a schematic structural diagram of a first sealing member of the atomizer shown in FIG. 2;
FIG. 8 is a schematic structural diagram of an atomization top base of the atomizer shown in FIG. 2;
FIG. 9 is a schematic structural diagram of a second sealing member of the atomizer shown in FIG. 2;
FIG. 10 is a schematic diagram of an exploded structure of an ultrasonic atomization chip assembly of the atomizer shown in FIG. 2;
FIG. 11 is a schematic structural diagram of first micropores in a microporous sheet of the ultrasonic atomization chip assembly shown in FIG. 10; and
FIG. 12 is a schematic diagram of an arrangement of micropores in a microporous sheet of the ultrasonic atomization chip assembly shown in FIG. 10.

### DETAILED DESCRIPTION

Specific implementations of the present disclosure are described in detail below with reference to the accompanying drawings. It should be understood that the specific implementations described herein are merely used to describe and explain the present disclosure, but are not intended to limit the present disclosure.

The terms used in the present disclosure are for the purpose of describing specific embodiments only and are not intended to limit the present disclosure. Unless otherwise defined, technical terms or scientific terms used in the present disclosure are to have general meanings understood by those of ordinary skill in the field to which the present disclosure belongs. "First", "second", and similar terms used in the specification and the claims of the present disclosure do not indicate any order, quantity or significance, but are used to only distinguish different components. Similarly, "one", "a", and similar terms also do not indicate a quantity limitation, but indicate that there is at least one. If only "one" is indicated, this is separately described. "Plurality of" or "several" indicates two or more. Unless otherwise specified, "front", "rear", "lower", "upper", and/or similar terms are merely for ease of description, and are not limited to one position or one spatial orientation. "Include", "including", or similar terms mean that elements or items appearing before the term cover elements or items listed after the term and equivalents thereof, but do not exclude other elements or items. "Connection", "link", or similar terms are not limited to a physical or mechanical connection, but may include an electrical connection, whether directly or indirectly. The singular forms "a", "the", and "this" used in the specification and the appended claims of the present disclosure are intended to include the plural forms as well, unless the context clearly indicates otherwise. It should be further understood that the term "and/or" used herein indicates and includes any or all possible combinations of one or more associated listed items.

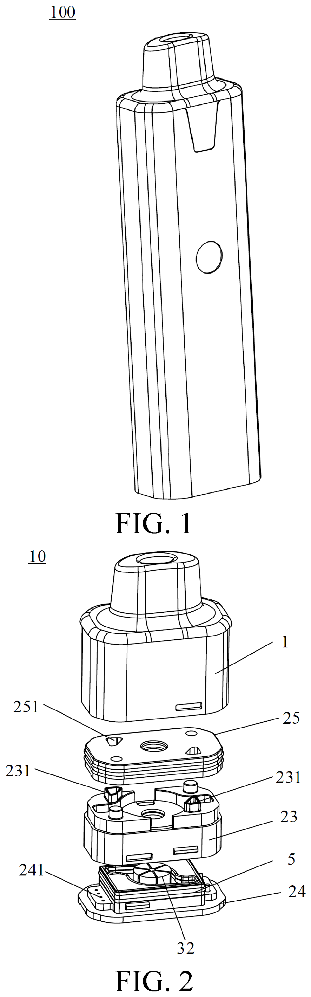

As shown in FIG. 1, an embodiment of the present disclosure provides an aerosol generating device 100. The aerosol generating device 100 includes an atomizer 10 according to any embodiment of the present disclosure and a power supply component (not shown in the figure).

The aerosol generating device 100 is used for atomizing an aerosol generating substrate to generate an aerosol for user inhalation. The aerosol generating substrate includes, but is not limited to, a medication, a nicotine-containing material, a nicotine-free material, and the like. In embodiments of the present disclosure, the aerosol generating substrate may be, for example, a liquid material having a plant (such as tobacco) as a main raw material and containing a corresponding aerosol forming agent and an aroma material.

The power supply component is electrically connected to the atomizer 10 and is used for supplying power to the atomizer 10. In embodiments of the present disclosure, the power supply component is electrically connected to the atomizer 10 in a detachable mounting manner, which can facilitate subsequent replacement and maintenance.

Those skilled in the art should understand that a type of the aerosol generating device 100 is not specifically limited in embodiments of the present disclosure. For example, the aerosol generating device 100 may be a device that needs to use the atomizer 10, such as a medical nebulizer, an air humidifier, or an electronic cigarette.

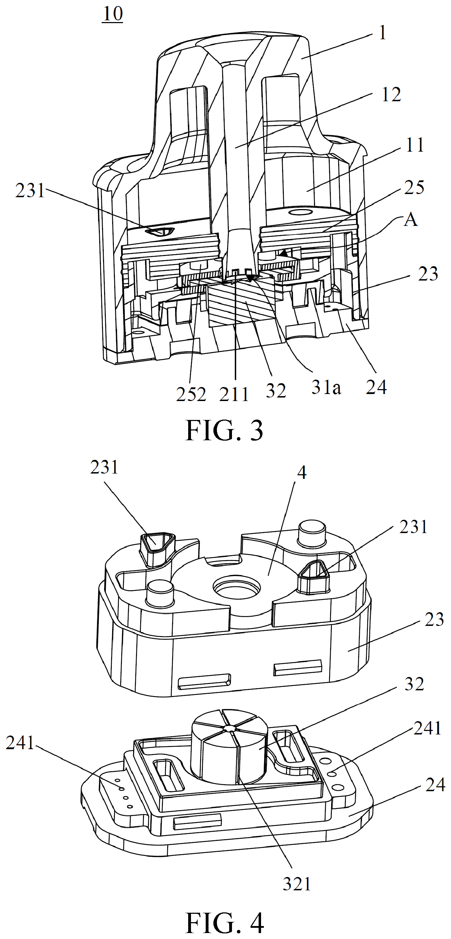

As shown in FIG. 2 and FIG. 3, an embodiment of the present disclosure provides an atomizer 10. The atomizer 10 includes a housing assembly 1, an atomization base 2, and an atomization core 3.

The housing assembly 1 is an external housing of the atomizer 10, and a liquid storage cavity 11 and an air outlet channel 12 are formed in the housing assembly. At least a part of the atomization base 2 is disposed in the housing assembly 1. The liquid storage cavity 11 is used for storing an aerosol generating substrate. In embodiments of the present disclosure, the liquid storage cavity 11 is located on the top side of the atomization base 2, the top of the atomization base 2 and the inner side wall of the housing assembly 1 define the liquid storage cavity 11, the air outlet channel 12 is located in the middle area inside the housing assembly 1, and the liquid storage cavity 11 is arranged around the air outlet channel 12.

For example, that at least a part of the atomization base 2 is disposed in the housing assembly 1 may refer to that a partial structure of the atomization base 2 is disposed in the housing assembly 1, or an entire structure of the atomization base 2 is disposed in the housing assembly 1.

The atomization core 3 is used for absorbing the aerosol generating substrate and atomizing the aerosol generating substrate. The atomization core 3 is disposed on the atomization base 2 and includes an ultrasonic atomization chip assembly 31. The ultrasonic atomization chip assembly 31 is arranged perpendicular to a top-bottom direction of the atomizer 10. The ultrasonic atomization chip assembly 31 can convert electrical energy into vibration energy, to generate ultrasonic waves through vibration, and atomize the aerosol generating substrate in an ultrasonic atomization manner, to generate an aerosol for user inhalation.

An electronic cigarette is used as an example. Compared with a conventional heat-not-burn manner, the ultrasonic atomization manner used in embodiments of the present disclosure is implemented at low atomization temperature, thus resulting in low release of aldehydes and ketones from the aerosol generating substrate, reducing release of harmful substances from the aerosol generating substrate, improving safety performance, and reducing hazards.

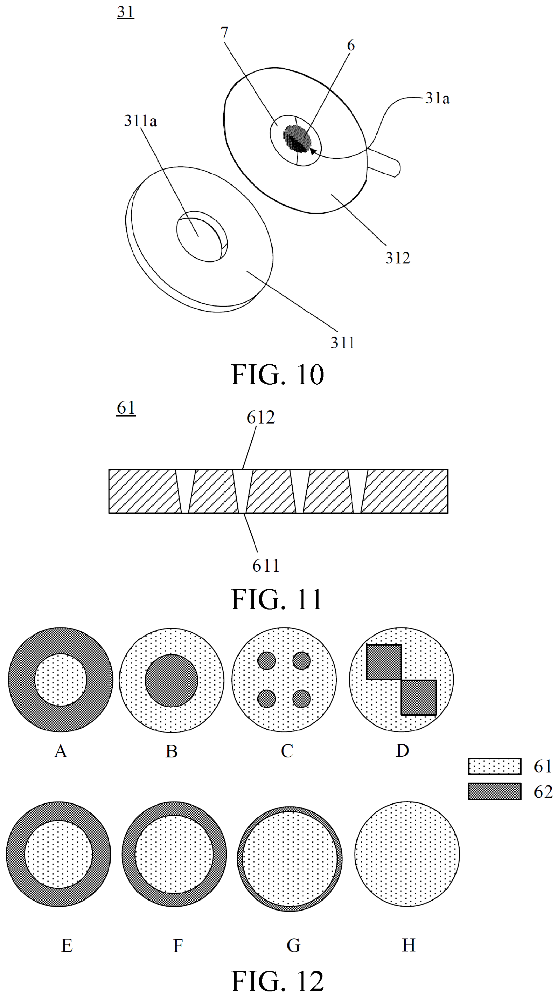

As shown in FIG. 10, in this embodiment of the present disclosure, the ultrasonic atomization chip assembly 31 is generally circular, and has the diameter of 10 mm to 15 mm (millimeters), preferably, 12 mm to 14 mm. The edge of the circular ultrasonic atomization chip assembly 31 is smooth and has no sharp corner, which can reduce damage to the ultrasonic atomization chip assembly 31 due to concentration of stress.

Of course, in some other embodiments, the ultrasonic atomization chip assembly 31 may alternatively be in any other suitable shape.

For example, the diameter of the ultrasonic atomization chip assembly 31 may be 10 mm, 11 mm, 12 mm, 13 mm, 14 mm, or 15 mm. The ultrasonic atomization chip assembly 31 has a suitable diameter, which can not only ensure that a sufficient quantity of aerosols are generated through atomization, but also prevent the ultrasonic atomization chip assembly 31 from being too large, which may hinder assembly into the atomizer.

As shown in FIG. 3, the ultrasonic atomization chip assembly 31 has an aerosol outlet area 31a used for discharging an aerosol. The aerosol outlet area 31a faces the top side of the atomizer 10, and the bottom end of the air outlet channel 12 covers the periphery of the aerosol outlet area 31a, to cause the aerosol generated through atomization to be discharged from the housing assembly 1 through the air outlet channel 12 for user inhalation.

In the related technology, typically, the atomizer is formed with an atomization cavity, the atomization cavity communicates with an air outlet channel, an aerosol generated through atomization first flows through the atomization cavity, then flows through the air outlet channel, and is finally discharged from the atomizer. A flow path of the aerosol is long, which increases a possibility of condensation of the aerosol, and further causes high loss of the aerosol. Typically, the atomization cavity includes a top wall, a bottom wall, and a side wall. Corners may exist at connections between the side wall and the top wall, as well as between the side wall and the bottom wall. This may increase a frequency of collision between the aerosol and each wall surface of the atomization cavity. In addition, a possibility of condensation of the aerosol at the corners is high. Therefore, loss of the aerosol may be further increased, which is not beneficial to user experience.

In embodiments of the present disclosure, the bottom end of the air outlet channel 12 directly covers the periphery of the aerosol outlet area 31a of the ultrasonic atomization chip assembly 31, and the aerosol outlet area 31a faces the top side of the atomizer 10. In this way, the aerosol generated by atomizing the aerosol generating substrate by the ultrasonic atomization chip assembly 31 does not flow through another area, and can directly flow upward to be discharged from the housing assembly 1 through the air outlet channel 12. In this way, the length of the flow path of the aerosol generated through atomization is reduced, and a frequency of collision of the aerosol is reduced, which can cause the aerosol to be discharged from the housing assembly 1 quickly for user inhalation. In addition, the possibility of condensation of the aerosol is reduced, and loss of the aerosol is reduced, which avoids a decrease in aroma and concentration caused by excessive loss of the aerosol, and improves user experience.

In addition, in such an upward atomization manner, the aerosol generating substrate needed to be pumped upward to the air outlet channel 12 by overcoming the gravity under vibration of the ultrasonic atomization chip assembly 31. In this way, when the atomizer 10 is not in operation, that is, when the ultrasonic atomization chip assembly 31 does not vibrate, it is difficult for the aerosol generating substrate in the liquid storage cavity 11 to enter the air outlet channel 12 through the ultrasonic atomization chip assembly 31, thereby reducing a possibility of liquid leakage. In addition, the aroma of the aerosol generating substrate in the liquid storage cavity 11 can be well retained.

The bottom end of the air outlet channel 12 directly covers the periphery of the aerosol outlet area 31a of the ultrasonic atomization chip assembly 31, which can reduce processing difficulty of the atomizer 10, and reduce production costs. In addition, this facilitates miniaturization and integration of the atomizer 10.

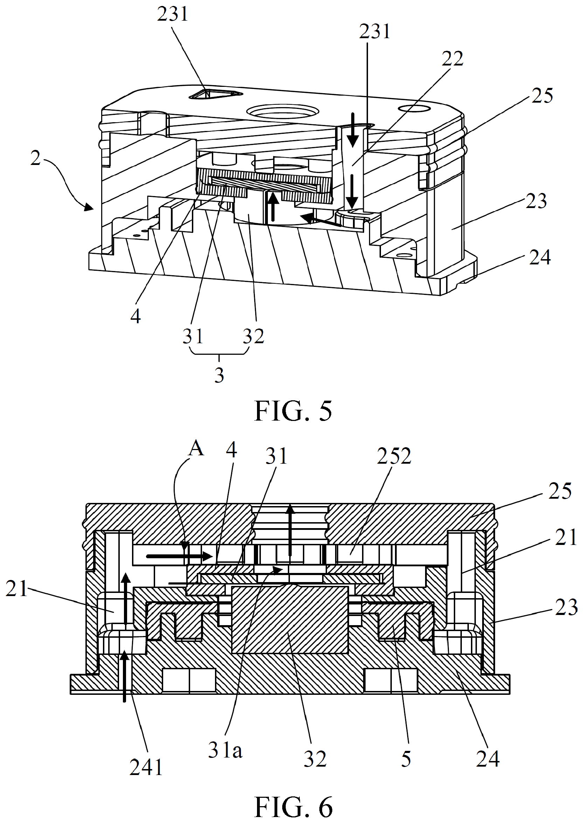

As shown in FIG. 5 and FIG. 6, the atomization base 2 is formed with an air inlet channel 21 and a liquid inlet channel 22. The air inlet channel 21 communicates with the outside, and the liquid inlet channel 22 communicates with the liquid storage cavity 11. As shown in FIG. 3, the side wall at the bottom end of the air outlet channel 12 is formed with at least one notch 211, and the air inlet channel 21 communicates with the air outlet channel 12 through the notch 211. The aerosol generating substrate in the liquid storage cavity 11 flows through the liquid inlet channel 22 to the ultrasonic atomization chip assembly 31 for ultrasonic atomization. An aerosol generated through atomization is discharged to the air outlet channel 12 through the aerosol outlet area 31a. In the air outlet channel 12, the aerosol is discharged, together with air flowing through the air inlet channel 21, from the housing assembly 1 for user inhalation.

In embodiments of the present disclosure, a plurality of (more than two) notches 211 are provided, and the plurality of notches 211 are arranged at intervals in a circumferential direction of the air outlet channel 12. The plurality of notches 211 are provided, which enables more air to enter the air outlet channel 12, to achieve smooth flow of the aerosol.

Those skilled in the art should understand that a number and a shape of the notch 211 are not specifically limited in embodiments of the present disclosure, and may be specifically set according to an actual size of the air outlet channel 12.

In some embodiments, at least one through hole may alternatively be formed in the side wall of the air outlet channel 12, and the air inlet channel 21 communicates with the air outlet channel 12 through the through hole.

In embodiments of the present disclosure, the air outlet channel 12 linearly extends in a top-bottom direction of the atomizer 10. The linear air outlet channel 12 has a simple structure and is easy to process, and meanwhile, has low airflow resistance. In addition, the linear air outlet channel 12 has no corner, which can reduce a possibility of collision between the aerosol and the airway wall of the air outlet channel 12, reduce loss of the aerosol caused by condensation of the aerosol, and improve user experience.

In embodiments of the present disclosure, the length of the air outlet channel 12 is not greater than 35 mm. Preferably, the length of the air outlet channel 12 is between 15 mm and 35 mm.

In the related technology, when the aerosol generating substrate is atomized in a heating atomization manner, the length of the air outlet channel of the atomizer needs to be set long, which can reduce the temperature of the aerosol, and prevent users from being burned due to excessively high temperature of the aerosol. However, the long air outlet channel may increase the possibility of condensation of the aerosol. Particularly, in a case that the viscosity of the aerosol generating substrate is low, the aerosol generated through atomization is more prone to condensation, resulting in high loss of the aerosol.

According to embodiments of the present disclosure, the aerosol generating substrate is atomized in the ultrasonic atomization manner, which does not cause the problem of excessively high temperature of the aerosol. Therefore, the length of the air outlet channel 12 can be shortened, to reduce a length of a moving path of the aerosol generated through atomization and a frequency of collision between the aerosol and the airway wall, thereby reducing loss of the aerosol caused by condensation, and improving user experience.

In addition, shortening the length of the air outlet channel 12 further facilitates the overall miniaturization of the atomizer 10.

For example, the length of the air outlet channel 12 may be 15 mm, 16 mm, 17 mm, 18 mm, 19 mm, 20 mm, 21 mm, 22 mm, 23 mm, 24 mm, 25 mm, 26 mm, 27 mm, 28 mm, 29 mm, 30 mm, 31 mm, 32 mm, 33 mm, 34 mm, or 35 mm.

In embodiments of the present disclosure, the equivalent diameter of the air outlet channel 12 is between 2.5 mm and 5.0 mm. The equivalent diameter refers to the corresponding diameter of a circle having a same sectional area. In embodiments of the present disclosure, when the cross-sectional shape of the air outlet channel 12 is a circle, the equivalent diameter of the air outlet channel 12 is the diameter of the circle.

For example, the equivalent diameter of the air outlet channel 12 may be 2.5 mm, 3.0 mm, 3.5 mm, 4.0 mm, 4.5 mm, or 5 mm. The equivalent diameter of the air outlet channel 12 is moderate, which not only can ensure smooth flow of a sufficient quantity of aerosols, but also can facilitate miniaturization and integration of the atomizer 10.

In embodiments of the present disclosure, the ultrasonic atomization chip assembly 31 is disposed on the atomization base 2. Compared with an aerosol output of an aerosol that is generated by the ultrasonic atomization chip assembly 31 in a bare state, the ultrasonic atomization chip assembly 31 is fixed in the atomization base 2, the aerosol generated by atomizing the aerosol generating substrate has clamping loss, and furthermore, when flowing through the air outlet channel 12 and finally discharged from the housing assembly 1, the aerosol has airway loss. The clamping loss is further described in detail below.

Table 1 shows experimental results obtained from tests on an aerosol output of an aerosol using the structure of the air outlet channel 12 provided in the foregoing embodiments. Specific parameters of the ultrasonic atomization chip assembly used in the experiment are as follows: the diameter is 14 mm, the thickness is 0.78 mm, an operating frequency is 130 kHz, an operating voltage is 80 Vpp, and the maximum central vibration area is 1.5 mm.

Example 1-1 to Example 1-5 respectively show clamping aerosol outputs and airway aerosol outputs that are obtained by adding test substrates with different viscosities to the ultrasonic atomization chip assembly.

The clamping aerosol output refers to an aerosol output of an aerosol that is generated through ultrasonic atomization performed by the ultrasonic atomization chip assembly 31 fixedly disposed on the atomization base 2. The airway aerosol output refers to an aerosol output of an aerosol generated through atomization as it flows through the air outlet channel 12 and is finally discharged from the housing assembly 1. Airway loss is defined as: airway loss = 100 × (clamping aerosol output - airway aerosol output) / clamping aerosol output.

**Table 1 Airway loss caused by ultrasonic atomization chip assembly**

| Number | Clamping aerosol output /(mg/puff) | Airway aerosol output/ (mg/puff) | Airway loss/% |
|---|---|---|---|
| Example 1-1 | 12.5 | 11.5 | 8.0 |
| Example 1-2 | 9.4 | 8.5 | 9.6 |
| Example 1-3 | 8.8 | 7.9 | 10.2 |
| Example 1-4 | 7.7 | 6.9 | 10.4 |
| Example 1-5 | 10.4 | 9.5 | 8.7 |

The unit mg/puff denotes milligrams/every three seconds.

In the related technology, airways loss after the aerosol flows through the air outlet channel is usually around 20%. However, as can be seen from Table 1, by using the structure of the air outlet channel 12 in embodiments of the present disclosure, the maximum airway loss is only approximately 10%, which indicates that loss of the aerosol is effectively reduced and the aerosol output of the aerosol is guaranteed.

As shown in FIG. 2 to FIG. 6, the atomization base 2 includes an atomization top base 23, an atomization bottom base 24, and a first sealing member 25. The atomization bottom base 24 is located on the bottom side of the atomization top base 23, and the first sealing member 25 is located on the top side of the atomization top base 23.

The atomization top base 23 is detachably connected to the atomization bottom base 24, and the first sealing member 25 is also detachably connected to the atomization top base 23. A detachable connection manner includes, but is not limited to, a threaded manner, a magnetic attraction manner, a snap-fit manner, or the like.

As shown in FIG. 3 and FIG. 4, a liquid inlet hole 231 is formed in the atomization top base 23, and the liquid inlet hole 231 communicates with the liquid storage cavity 11 and the liquid inlet channel 22.

For example, a plurality of liquid inlet holes 231 are provided on the atomization top base 23, that is, a plurality of liquid inlet channels 22 are provided in the atomization base 2, and each liquid inlet channel 22 is connected to a different position of the liquid storage cavity 11. In this way, the aerosol generating substrate at different positions of the liquid storage cavity 11 can be quickly guided by the plurality of liquid inlet holes 231 and the plurality of liquid inlet channels 22 to the atomization core 3, which allows smooth liquid discharge of the aerosol generating substrate, thereby achieving rapid atomization and meeting user requirements. In addition, this can further avoid occurrence of a case in which no aerosol generating substrate is delivered to the atomization core 3 when one liquid inlet channel 22 is blocked, thereby preventing the atomization core 3 from being damaged due to dry heating and improving the service life of the atomization core 3.

In embodiments of the present disclosure, two liquid inlet holes 231 are provided, and the two liquid inlet holes 231 are respectively located on two obliquely opposite sides of the atomization top base 23. That is, two liquid inlet channels 22 are formed in the atomization base 2, and the two liquid inlet channels 22 communicate with different positions of the liquid storage cavity 11.

Of course, in some other embodiments, a number of liquid inlet holes 231 and a number of liquid inlet channels 22 may be one, or may be more (more than two). The number of the two components is not specifically limited in embodiments of the present disclosure, and may be specifically set according to a size of the atomizer 10 and processing difficulty.

The first sealing member 25 is used for sealing the liquid storage cavity 11, and the first sealing member 25 is formed with an avoidance notch 251, and the avoidance notch is used for avoiding the liquid inlet hole 231. For example, the first sealing member 25 may be made of a material such as silicone. A material of the first sealing member 25 is not specifically limited in embodiments of the present disclosure.

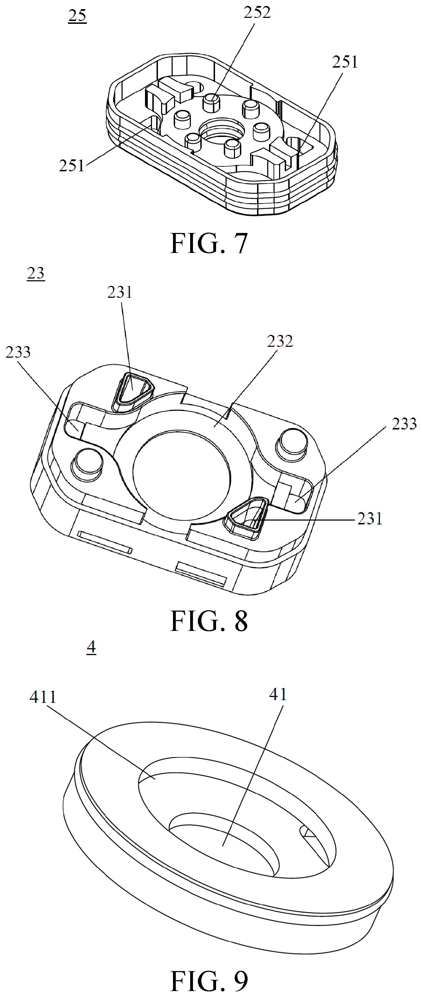

As shown in FIG. 7 and FIG. 8, a bottom surface of the first sealing member 25 is formed with a plurality of convex points 252, the atomization top base 23 is formed with a groove 232, the ultrasonic atomization chip assembly 31 is disposed in the groove 232, and the first sealing member 25 abuts against the ultrasonic atomization chip assembly 31 through the convex points 252, to fix the ultrasonic atomization chip assembly 31 in the groove 232.

When disposed on the atomization base 2, the ultrasonic atomization chip assembly 31 needs to be fixed. In the related technology, the ultrasonic atomization chip assembly is usually clamped between the atomization top base and the atomization bottom base. A contact area between a clamping surface and the ultrasonic atomization chip assembly is large, which affects vibration of the ultrasonic atomization chip assembly to some extent, thereby affecting an atomization effect of the ultrasonic atomization chip assembly.

However, in embodiments of the present disclosure, the ultrasonic atomization chip assembly 31 is fixed between the atomization top base 23 and the first sealing member 25, and the first sealing member 25 abuts against the ultrasonic atomization chip assembly 31 through the convex points 252. In this way, a contact area between the first sealing member 25 and the ultrasonic atomization chip assembly 31 can be reduced while fixing is achieved, thereby reducing impact on operation of the ultrasonic atomization chip assembly 31 and achieving a good atomization effect of the ultrasonic atomization chip assembly 31.

In embodiments of the present disclosure, the convex point 252 may be, for example, cylindrical. In some other embodiments, the convex point 252 may be, for another example, annular. A shape of the convex point 252 is not specifically limited in embodiments of the present disclosure, provided that a contact area with the ultrasonic atomization chip assembly 31 can be reduced, and the ultrasonic atomization chip assembly 31 can be fixed.

The convex points 252 and the first sealing member 25 are formed into an integrated structure, and are both made of flexible materials such as silicone. In this way, soft positioning of the ultrasonic atomization chip assembly 31 can be achieved and impact on functionality of the ultrasonic atomization chip assembly 31 is further reduced. In addition, the integrated structure can reduce a number of parts and reduce assembly difficulty. Of course, in some other embodiments, the convex points 252 and the first sealing member 25 may alternatively be assembled as separate structures.

As shown in FIG. 7, the plurality of convex points 252 are uniformly arranged on the bottom surface of the first sealing member 25 at intervals, and an arrangement shape is generally circular, which matches the shape of the ultrasonic atomization chip assembly 31. In this way, the ultrasonic atomization chip assembly 31 can be well fixed while the contact area is reduced, thereby reducing occurrence of situations such as flipping and shifting of the ultrasonic atomization chip assembly 31. In some other embodiments, when the ultrasonic atomization chip assembly 31 is in another shape, the arrangement shape of the plurality of convex points 252 may also be correspondingly changed. The number and the arrangement shape of the convex points 252 are not specifically limited in embodiments of the present disclosure.

The groove 232 can limit the ultrasonic atomization chip assembly 31, thereby well fixing the ultrasonic atomization chip assembly 31. In addition, this can facilitate positioning of the ultrasonic atomization chip assembly 31 during assembly, thereby reducing assembly difficulty and reducing production costs.

Table 2 shows experimental results obtained from tests on an aerosol output of an aerosol after the ultrasonic atomization chip assembly 31 is fixed through the structure of the convex points 252 in the foregoing embodiments. Specific parameters of the ultrasonic atomization chip assembly used in the experiment are as follows: the diameter is 14 mm, the thickness is 0.78 mm, an operating frequency is 130 kHz, an operating voltage is 80 Vpp, and the maximum central vibration area is 1.5 mm.

Example 2-1 to Example 2-5 respectively show bare-chip aerosol outputs and clamping aerosol outputs that are obtained by adding test substrates with different viscosities to the foregoing ultrasonic atomization chip assembly.

The bare-chip aerosol output refers to an aerosol output of an aerosol that is generated through ultrasonic vibration of the ultrasonic atomization chip assembly when the ultrasonic atomization chip assembly 31 is not clamped or fixed in any manner, and is only horizontally placed on an experimental platform and kept suspended. The clamping aerosol output refers to an aerosol output of an aerosol that is generated through ultrasonic atomization performed by the ultrasonic atomization chip assembly 31 fixedly disposed on the atomization base 2 through the foregoing convex points 252. Clamping loss is defined as follows: clamping loss = 100 × (bare-chip aerosol output - clamping aerosol output) / bare-chip aerosol output.

**Table 2 Clamping loss caused by ultrasonic atomization chip assembly**

| Number | Bare-chip aerosol output /(mg/puff) | Clamping aerosol output/ (mg/puff) | Clamping loss/% |
|---|---|---|---|
| Example 2-1 | 14.0 | 12.5 | 10.7 |
| Example 2-2 | 10.3 | 9.4 | 8.7 |
| Example 2-3 | 9.7 | 8.8 | 8.8 |
| Example 2-4 | 8.4 | 7.7 | 8.3 |
| Example 2-5 | 11.5 | 10.4 | 9.6 |

As can be seen from Table 2, in embodiments of the present disclosure, when the ultrasonic atomization chip assembly 31 is fixed through the structure of the convex points 252, the maximum clamping loss is only approximately 10%, which indicates that the clamping loss can be effectively reduced and the aerosol output of the aerosol is guaranteed.

As shown in FIG. 6, an arrow in the figure indicates a flow direction of air. A spacing area A is formed between the bottom surface of the first sealing member 25 and the top surface of the ultrasonic atomization chip assembly 31, and the convex point 252 is located in the spacing area A. The atomization top base 23 is formed with at least one communication groove 233, the atomization bottom base 24 is formed with at least one air inlet hole 241, the communication groove 233 communicates with the air inlet hole 241 and the spacing area A, and the air inlet hole 241, the communication groove 233, and the spacing area A define at least a part of the air inlet channel 21.

For example, a plurality of the air inlet holes 241 may be provided, and a plurality of the communication grooves 233 may also be provided and respectively communicate with the plurality of air inlet holes 241, to form a plurality of air inlet channels 21. This allows more air to quickly enter the atomizer 10 through the plurality of air inlet channels 21, to achieve smooth flow of the aerosol.

A number of air inlet holes 241 and a number of communication grooves 233 are not specifically limited in embodiments of the present disclosure, and may be one, two, or more.

As shown in FIG. 9, the atomizer 10 includes a second sealing member 4. The second sealing member 4 is formed with a first through hole 41, an annular groove 411 is formed in the hole wall of the first through hole 41, the second sealing member 4 surrounds the ultrasonic atomization chip assembly 31, and the edge of the ultrasonic atomization chip assembly 31 is embedded into the annular groove 411.

The second sealing member 4 generally wraps the ultrasonic atomization chip assembly 31, to prevent a leaking aerosol generating substrate from affecting the performance and atomization effect of the ultrasonic atomization chip assembly 31 in a case of liquid leakage.

As shown in FIG. 2 and FIG. 6, the atomizer 10 includes a third sealing member 5. The third sealing member 5 is located between the atomization top base 23 and the atomization bottom base 24, and surrounds a connection position between the atomization top base 23 and the atomization bottom base 24, to prevent the leaking aerosol generating substrate from flowing to a power supply component through the atomization bottom base 24 in a case of liquid leakage, thereby avoiding damage to the power supply component.

The second sealing member 4 and the third sealing member 5 may also be made of materials such as silicone. Materials of the second sealing member 4 and the third sealing member 5 are not specifically limited in embodiments of the present disclosure.

As shown in FIG. 10, the ultrasonic atomization chip assembly 31 includes a piezoelectric ceramic sheet 311 and a microporous sheet 312. The microporous sheet 312 is superposed on the bottom side of the piezoelectric ceramic sheet 311 and is connected to the piezoelectric ceramic sheet 311, a second through hole 311a is formed in the middle area of the piezoelectric ceramic sheet 311, micropores 6 are formed in the microporous sheet 312, the micropores 6 are form in a portion, located in the second through hole 311a, of the microporous sheet 312, and the area of the microporous sheet 312 in which the micropores 6 are formed constitutes an aerosol outlet area 31a. When the atomizer 10 is in operation, the piezoelectric ceramic sheet 311 undergoes mechanical deformation with changes of a voltage and a frequency. In this way, during vibration, the aerosol generating substrate in the liquid inlet channel 22 is pumped to the air outlet channel 12 through the micropores 6 in the microporous sheet 312 after being atomized.

For example, the microporous sheet 312 may be adhered to the bottom side of the piezoelectric ceramic sheet 311 through a conductive adhesive.

In embodiments of the present disclosure, the thickness of the piezoelectric ceramic sheet 311 is between 0.4 mm and 0.8 mm, and/or the diameter of the piezoelectric ceramic sheet 311 is between 12 mm and 14 mm.

For example, the thickness of the piezoelectric ceramic sheet 311 may be 0.4 mm, 0.5 mm, 0.6 mm, 0.7 mm, or 0.8 mm; and the diameter of the piezoelectric ceramic sheet 311 may be 12 mm, 13 mm, or 14 mm. The thickness and the diameter of the piezoelectric ceramic sheet 311 are moderate. In this way, no excessive space in the atomizer 10 is occupied while a good atomization effect is guaranteed, which is beneficial to miniaturization of the device.

For example, a material of the piezoelectric ceramic sheet 311 may be one of lead zirconate titanate, barium carbonate, and potassium sodium niobate. Of course, in some other embodiments, the piezoelectric ceramic sheet 311 may alternatively be made of any other suitable material.

In embodiments of the present disclosure, the diameter of the area of the microporous sheet 312 in which the micropores 6 are formed is between 1 mm and 4 mm.

For example, the diameter of the area of the microporous sheet 312 in which the micropores 6 are formed may be 1 mm, 2 mm, 3 mm, or 4 mm.

In embodiments of the present disclosure, the thickness of the microporous sheet 312 is between 0.02 mm and 0.1 mm, and/or the diameter of the microporous sheet 312 is between 12 mm and 14 mm.

For example, the thickness of the microporous sheet 312 may be 0.02 mm, 0.03 mm, 0.04 mm, 0.05 mm, 0.06 mm, 0.07 mm, 0.08 mm, 0.09 mm, or 0.1 mm; and the diameter of the microporous sheet 312 may be 12 mm, 13 mm, or 14 mm. The thickness and the diameter of the microporous sheet 312 are moderate. In this way, ultrasonic vibration of the piezoelectric ceramic sheet 311 is not affected.

For example, a material of the microporous sheet 312 may be one of stainless steel, nickel-cobalt alloy, titanium alloy, copper alloy, and palladium-nickel alloy. These materials have high hardness. Therefore, even when the microporous sheet 312 vibrates together with the piezoelectric ceramic sheet 311, the microporous sheet can maintain its shape well and is not easily deformed.

Of course, in some other embodiments, the microporous sheet 312 may alternatively be made of any other suitable material.

In embodiments of the present disclosure, a number of micropores 6 is between 500 to 4,000, and a spacing distance between any two adjacent micropores 6 is between 40 µm and 100 µm (micrometers).

For example, the number of micropores 6 may be 500, 1000, 1500, 2000, 2500, 3000, 3500, or 4000; and the spacing distance between two micropores 6 may be 40 µm, 50 µm, 60 µm, 70 µm, 80 µm, 90 µm, or 100 µm.

The number and the spacing distance between the micropores 6 in the microporous sheet 312 are moderate. In this way, successful pumping and atomization of the aerosol generating substrate can be guaranteed, and volatilization of the aroma of the aerosol generating substrate when the atomizer 10 is not in operation can be reduced, thereby reducing a possibility of liquid leakage.

In embodiments of the present disclosure, the micropores 6 in the microporous sheet 312 are formed through a laser perforation process. The laser perforation process has high precision and a high speed, and is highly flexible and adaptable, which can achieve processing of a plurality of pores of different shapes and sizes. In addition, the laser perforation process is a non-contact processing manner, does not cause mechanical damage to a to-be-perforated structure, and can guarantee integrity and quality of the structure.

Of course, in some other embodiments, the micropores 6 may alternatively be processed through any other suitable perforation process such as electroforming perforation or etching perforation.

In the related technology, an electronic cigarette is used as an example, an aerosol that is generated by performing ultrasonic atomization on the aerosol generating substrate in an ultrasonic vibration manner usually has large particle size, and excessive aerosols with large particle size cause a large local concentration of nicotine, which results in high harshness and affects user experience. Particularly, when water is used instead of PG/VG (propylene glycol/glycerol) as a primary substrate for the aerosol generating substrate, the harshness to the throat is greater. Therefore, the particle size of the aerosol generated through atomization usually needs to be less than 2.5 µm to provide users with good inhalation experience. The particle size of an aerosol that is generated by atomizing the aerosol generating substrate in a conventional heating manner can easily fall within the particle size range. However, at excessively high heating temperature, propylene glycol and glycerol serving as substrates in the aerosol generating substrate are prone to decomposition, so as to produce some harmful substances. In addition, a conductive heating structure for heating in the atomization core often operates at high temperature, which can accelerate release of some heavy metals and may pose hazards to users. The foregoing situation may be well avoided by a low-temperature atomization manner such as ultrasonic vibration. However, in the related technology, the aerosol generated by atomizing the aerosol generating substrate in an ultrasonic vibration manner usually has large particle size, which makes it difficult to fall within the foregoing particle size range, thereby affecting inhalation experience of users.

In view of this, the structure of the micropores 6 is improved in embodiments of the present disclosure. As shown in FIG. 11, the micropores 6 include first micropores 61. The first micropores 61 form a first aerosol outlet 611 on the surface on the side, facing the piezoelectric ceramic sheet 311, of the microporous sheet 312, and the pore size of the first aerosol outlet 611 is not greater than 2.0 µm. Preferably, the pore size of the first aerosol outlet 611 is between 1.1 µm and 2.0 µm.

For example, the pore size of the first aerosol outlet 611 may be 1.1 µm, 1.2 µm, 1.3 µm, 1.4 µm, 1.5 µm, 1.6 µm, 1.7 µm, 1.8 µm, 1.9 µm, or 2.0 µm.

Table 3 shows experimental results obtained from bare-chip ultrasonic atomization tests using microporous sheets 312 having first aerosol outlets 611 with different pore sizes. Specific parameters of the ultrasonic atomization chip assemblies used in the experiment are as follows: the diameter is 14 mm, the thickness is 0.78 mm, an operating frequency is 130 kHz, an operating voltage is 80 Vpp, the maximum central vibration area is 1.5 mm, and the substrate viscosity is 4 cP (centipoise). Specific parameters of the microporous sheets of the ultrasonic atomization chip assemblies are as follows: the diameter is 14 mm, the thickness is 0.05 mm, the perforation area is 2 mm, the first liquid inlet pore size is 40 µm, the number of micropores is 2,000, and the porosity is 85%.

**Table 3 Aerosol particle sizes corresponding to different first aerosol outlet pore sizes of microporous sheets**

| Number | First aerosol outlet pore size/µm | Aerosol particle size/µm |
|---|---|---|
| Example 3-1 | 1.1 | 1.5 |
| Example 3-2 | 1.2 | 1.7 |
| Example 3-3 | 1.4 | 1.9 |
| Example 3-4 | 1.6 | 2.1 |
| Example 3-5 | 1.7 | 2.2 |
| Example 3-6 | 1.8 | 2.3 |
| Example 3-7 | 2.0 | 2.5 |
| Comparative example 3-1 | 2.5 | 3.0 |

As can be seen from Table 3, when the pore size of the first aerosol outlet 611 is not greater than 2.0 µm, the maximum particle size of the aerosol generated through atomization is not greater than 2.5 µm. Therefore, by improving the pore size of the aerosol outlet of the micropores 6 in the microporous sheet 312, the particle size of the aerosol generated through atomization can be reduced, thereby improving user experience.

In addition, when the atomizer 10 is not in operation, and the pore size of the first aerosol outlet 611 is moderate, volatilization of the aroma of the aerosol generating substrate can be reduced, and a possibility of liquid leakage is reduced, thereby further improving user experience.

However, when the pore size of the aerosol outlet is decreased, an aerosol area is also reduced, thereby reducing an aerosol output of an aerosol. An insufficient aerosol output of the atomizer 10 also affects user experience. Typically, the aerosol output of the aerosol generated through atomization performed by the ultrasonic atomization chip assembly 31 is further affected by clamping loss and airway loss. Therefore, the aerosol output of the aerosol generated by the ultrasonic atomization chip assembly 31 in a bare state needs to be at least greater than 8 mg/puff, preferably greater than 10 mg/puff, to guarantee that a final aerosol output of the aerosol discharged from the atomizer 10 can meet user requirements.

To meet both requirements of a small aerosol particle size and a sufficient aerosol output of an aerosol, the structure of the first micropores 61 is further improved in embodiments of the present disclosure. The first micropores 61 form a first liquid inlet 612 on the surface on the side, facing away from the piezoelectric ceramic sheet 311, of the microporous sheet 312, and the pore size of the first liquid inlet 612 is preferably between 20 µm and 50 µm.

For example, the pore size of the first liquid inlet 612 may be 20 µm, 25 µm, 30 µm, 35 µm, 40 µm, 45 µm, or 50 µm.

Table 4 shows experimental results obtained from bare-chip ultrasonic atomization tests using bare microporous sheets 312 having first aerosol outlets 611 with the pore size of 1.7 µm and first liquid inlets 612 with different pore sizes. Specific parameters of the ultrasonic atomization chip assemblies used in the experiment are as follows: the diameter is 14 mm, the thickness is 0.78 mm, an operating frequency is 130 kHz, an operating voltage is 80 Vpp, the maximum central vibration area is 1.5 mm, and the substrate viscosity is 4 cP. Specific parameters of the microporous sheets of the ultrasonic atomization chip assemblies are as follows: the diameter is 14 mm, the thickness is 0.05 mm, the perforation area is 2 mm, the first aerosol outlet pore size is 1.7 µm, the number of micropores is 2,000, and the porosity is 85%.

**Table 4 Aerosol outputs corresponding to different first liquid inlet pore sizes of microporous sheets**

| Number | First liquid inlet pore size/µm | Aerosol output/(mg/puff) |
|---|---|---|
| Example 4-1 | 10 | 9.3 |
| Example 4-2 | 20 | 10.8 |
| Example 4-3 | 30 | 12.5 |
| Example 4-4 | 40 | 14.0 |
| Example 4-5 | 50 | 13.2 |
| Example 4-6 | 60 | 12.9 |
| Example 4-7 | 70 | 12.4 |

As can be seen from Table 4, when the pore size of the first liquid inlet 612 is between 20 µm and 50 µm, the aerosol output of the aerosol is above 8 mg/puff. In addition, as the pore size of the first liquid inlet 612 increases, the aerosol output of the aerosol is first increased and then decreased. When the pore size of the first liquid inlet 612 is 40 µm, the maximum aerosol output of the aerosol is achieved.

In embodiments of the present disclosure, the pore sizes of the first aerosol outlet 611 and the first liquid inlet 612 of the first micropores 61 are improved, to ensure that the pore size of the first aerosol outlet 611 is not greater than 2 pm, preferably between 1.1 µm and 2 µm, and the pore size of the first liquid inlet 612 is preferably between 20 µm and 50 µm. In this way, a sufficient aerosol output of the aerosol can be guaranteed while the aerosol generated through atomization performed by the ultrasonic atomization chip assembly 31 has small enough particle size, thereby significantly improving user experience.

In embodiments of the present disclosure, a shape of the first micropore 61 may be tapered, rectangular, or cylindrical. As long as the pore sizes of the first aerosol outlet 611 and the first liquid inlet 612 of the first micropores 61 fall within the foregoing pore size ranges, the first micropores 61 may be in any other suitable shape.

As shown in FIG. 11, preferably, the shape of the first micropore 61 is tapered. In this way, when the aerosol generating substrate is extruded out through the tapered first micropores 61, if the aerosol generating substrate is closer to the aerosol outlet area 31a, the aerosol generating substrate is further extruded, to allow the particle size of the aerosol that is finally discharged through the first aerosol outlet 611 to be smaller and the aerosol to be discharged more quickly.

In embodiments of the present disclosure, a vibration frequency of the ultrasonic atomization chip assembly 31 during operation is between 100 kHz and 180 kHz (kilohertz), an effective electro-mechanical coupling coefficient is greater than 0.38, a resonant impedance is less than 50 Ω (ohm), and a driving voltage is between 60 Vpp and 120 Vpp (peak-to-peak value).

For example, the vibration frequency of the ultrasonic atomization chip assembly 31 may be 100 kHz, 110 kHz, 120 kHz, 130 kHz, 140 kHz, 150 kHz, 160 kHz, 170 kHz, or 180 kHz.

Table 5 shows experimental results obtained from bare-chip ultrasonic atomization tests using the ultrasonic atomization chip assembly 31 at different vibration frequencies. Specific parameters of the ultrasonic atomization chip assembly used in the experiment are as follows: the diameter is 14 mm, the thickness is 0.78 mm, an operating voltage is 80 Vpp, the maximum central vibration area is 1.5 mm, and the substrate viscosity is 4 cP. Specific parameters of the microporous sheet of the ultrasonic atomization chip assembly are as follows: the diameter is 14 mm, the thickness is 0.05 mm, the perforation area is 2 mm, the first aerosol outlet pore size is 1.7 µm, the first liquid inlet pore size is 40 µm, the number of micropores is 2,000, and the porosity is 85%.

**Table 5 Aerosol outputs corresponding to ultrasonic atomization chip assembly at different vibration frequencies**

| Number | Vibration frequency/kHz | Aerosol output/(mg/puff) |
|---|---|---|
| Comparative example 5-1 | 17 | < 0.1 |
| Comparative example 5-2 | 79 | 2.1 |
| Example 5-1 | 104 | 8.3 |
| Example 5-2 | 123 | 11.7 |
| Example 5-3 | 130 | 14.0 |
| Example 5-4 | 165 | 11.2 |

As can be seen from Table 5, when the vibration frequencies of the ultrasonic atomization chip assembly 31 are 104 kHz, 123 kHz, 130 kHz, and 165 kHz, the aerosol outputs of the aerosols generated through atomization can all be guaranteed to be greater than 8 mg/puff. That is, the moderate vibration frequency of the ultrasonic atomization chip assembly 31 can guarantee that the aerosol output of the aerosol generated through atomization meets user requirements, thereby improving user experience.

In addition to the parameters of the ultrasonic atomization chip assembly 31, the substrate viscosity of the aerosol generating substrate may also affect the aerosol output of the aerosol generated through atomization.

Table 6 shows experimental results obtained from bare-chip ultrasonic atomization tests conducted at 25°C using aerosol generating substrates with different substrate viscosities. Specific parameters of the ultrasonic atomization chip assembly used in the experiment are as follows: the diameter is 14 mm, the thickness is 0.78 mm, an operating voltage is 80 Vpp, an operating frequency is 130 kHz, and the maximum central vibration area is 1.5 mm. Specific parameters of the microporous sheet of the ultrasonic atomization chip assembly are as follows: the diameter is 14 mm, the thickness is 0.05 mm, the perforation area is 2 mm, the first aerosol outlet pore size is 1.7 µm, the first liquid inlet pore size is 40 µm, the number of micropores is 2,000, and the porosity is 85%.

**Table 6 Aerosol outputs corresponding to different substrate viscosities**

| Number | Substrate viscosity/cP | Aerosol output/(mg/puff) |
|---|---|---|
| Example 6-1 | 1.0 | 18.3 |
| Example 6-2 | 2.2 | 16.5 |
| Example 6-3 | 4.0 | 14.0 |
| Example 6-4 | 6.5 | 12.0 |
| Example 6-5 | 7.5 | 9.9 |
| Example 6-6 | 8.4 | 9.2 |
| Example 6-7 | 10.0 | 8.1 |
| Comparative example 6-1 | 14.8 | 5.5 |
| Comparative example 6-2 | 21.7 | 1.3 |

As can be seen from Table 6, when the substrate viscosity is less than or equal to 10 cP, the aerosol output of the aerosol can be guaranteed to be greater than 8 mg/puff. Therefore, in embodiments of the present disclosure, a low-viscosity substrate, such as water or ethanol, is preferably selected as the primary substrate of the aerosol generating substrate. However, those skilled in the art should understand that the substrate viscosity of the used aerosol generating substrate is not specifically limited in embodiments of the present disclosure.

For example, when substrate viscosities of some aerosol generating substrates are high, the substrate viscosities of the aerosol generating substrates may be reduced through preheating before the aerosol generating substrates enter the ultrasonic atomization chip assembly 31, to guarantee the final aerosol output of the aerosol generated through atomization.

An example in which the aerosol generating device is an electronic cigarette is used. Excessive aerosols with large particle size can cause high harshness to users and negatively impact inhalation experience of the users. However, a small quantity of aerosols with the large particle size can enhance sweetness and aroma, thereby improving sensory attributes.

In embodiments of the present disclosure, the micropores 6 formed in the microporous sheet 312 further include second micropores 62. The second micropores 62 form a second aerosol outlet on the surface on the side, facing the piezoelectric ceramic sheet 311, of the microporous sheet 312, and the pore size of the second aerosol outlet is between 5 µm and 20 µm. To guarantee a sufficient aerosol output of the aerosol, the pore size of a second liquid inlet formed by the second micropores 62 on the surface on the side, facing away from the piezoelectric ceramic sheet 311, of the microporous sheet 312 is also between 20 µm and 50 µm.

For example, the pore size of the second aerosol outlet may be 5 µm, 6 µm, 7 µm, 8 µm, 9 µm, 10 µm, 11 µm, 12 µm, 13 µm, 14 µm, 15 µm, 16 µm, 17 µm, 18 µm, 19 µm, or 20 µm; and the pore size of the second liquid inlet may be 20 µm, 25 µm, 30 µm, 35 µm, 40 µm, 45 µm, or 50 µm.

In this way, not only the first micropores 61 capable of discharging an aerosol with the small particle size are formed, but also the second micropores 62 capable of discharging an aerosol with the large particle size are formed in the microporous sheet 312. In this way, the sweetness and the aroma can be properly improved while low throat harshness is guaranteed, thereby providing users with good inhalation experience.

As shown in FIG. 12, a light-colored part represents a distribution area of the first micropores 61, and a dark-colored part represents a distribution area of the second micropores 62.

For example, as shown in A, E, F, and G in FIG. 12, the first micropores 61 may be concentrated in the central area of the microporous sheet 312, and the second micropores 62 are distributed around the first micropores 61.

For another example, as shown in B in FIG. 12, the second micropores 62 may be concentrated in the central area of the microporous sheet 312, and the first micropores 61 are distributed around the second micropores 62.

For still another example, as shown in C in FIG. 12, the second micropores 62 may be distributed in a plurality of circular areas, the plurality of circular areas are arranged at intervals in a circumferential direction in the central area of the microporous sheet 312, and the first micropores 61 are distributed in another area of the microporous sheet 312 other than the circular areas.

For still another example, as shown in D in FIG. 12, the second micropores 62 may be distributed in two rectangular areas, the two rectangular areas are symmetrically arranged along the diameter of the microporous sheet 312, and the first micropores 61 are distributed in another area of the microporous sheet 312 other than the rectangular areas.

For still another example, as shown in H in FIG. 12, only the first micropores 61 are formed in the microporous sheet 312, and no second micropores 62 are formed.

Those skilled in the art should understand that the first micropores 61 and the second micropores 62 may alternatively be distributed on the microporous sheet 312 in any other manner. Distribution manners of the first micropores 61 and the second micropores 62 on the microporous sheet 312 are not specifically limited in embodiments of the present disclosure.

In embodiments of the present disclosure, a ratio of a number of first micropores 61 to a number of second micropores 62 ranges from 7: 1 to 10: 1. Preferably, the ratio of the number of first micropores 61 to the number of second micropores 62 is 9: 1, that is, the number of first micropores 61 accounts for 90% of a total number of micropores 6. In this way, aroma and sweetness can be improved while low harshness is guaranteed, thereby improving inhalation experience of users.

Of course, in some other embodiments, ratios of the number of first micropores 61 and the number of the second micropores 62 to the total number of micropores 6 may alternatively be changed adaptively.

Through different arrangement combinations of parameters, such as the distribution manner, the distribution area, and the pore size, of the first micropores 61 and the second micropores 62 in the microporous sheet 312, multi-dimensional regulation of sensory attributes of the aerosol generated through atomization, such as aroma, sweetness, harshness, and satisfaction, can be achieved.

**Table 7 Effects of different micropore arrangement solutions and aerosol outlet pore sizes on sensory attributes**

| Number | Micropore arrangement solution | First aerosol outlet pore size/µm | Second aerosol outlet pore size/µm | Sensory attribute evaluation result | | | |
|---|---|---|---|---|---|---|---|
| | | | | Aroma | Sweetness | Harshness | Satisfaction |
| Example 7-1 | A | 1.7 | 7 | Rich | Excessively sweet | High | Strong |
| Example 7-2 | B | 1.7 | 7 | Medium | Excessively sweet | Moderate | Moderate |
| Example 7-3 | C | 1.7 | 7 | Medium | Sweet | Moderate | Moderate |
| Example 7-4 | D | 1.7 | 7 | Rich | Excessively sweet | Moderate | Strong |
| Example 7-5 | E | 1.7 | 7 | Rich | Sweet | Moderate | Moderate |
| Example 7-6 | F | 1.7 | 7 | Medium | Sweet | Moderate | Strong |
| Example 7-7 | G | 1.7 | 7 | Medium | Slightly sweet | Moderate | Strong |
| Example 7-8 | H | 1.7 | 7 | Medium | Slightly sweet | Soft | Moderate |
| Example 7-9 | G | 2.0 | 5 | Medium | Slightly sweet | High | Strong |
| Example 7-10 | G | 1.7 | 10 | Rich | Excessively sweet | High | Strong |
| Example 7-11 | G | 1.7 | 15 | Rich | Excessively sweet | High | Strong |
| Example 7-12 | G | 1.5 | 15 | Rich | Excessively sweet | Moderate | Strong |
| Example 7-13 | G | 1.2 | 20 | Rich | Excessively sweet | Moderate | Strong |

The micropore arrangement solutions in Table 7 correspond to eight different distribution manners of the first micropores 61 and the second micropores 62 in the microporous sheet 312 in A to H in FIG. 12. Specific parameters of the ultrasonic atomization chip assemblies used in the experiment are as follows: the diameter is 14 mm, the thickness is 0.78 mm, an operating frequency is 130 kHz, an operating voltage is 80 Vpp, the maximum central vibration area is 1.5 mm, and the substrate viscosity is 4 cP. Specific parameters of the microporous sheets of the ultrasonic atomization chip assemblies are as follows: the diameter is 14 mm, the thickness is 0.05 mm, the first liquid inlet pore size is 40 µm, the second liquid inlet pore size is 40 µm, the total number of micropores is 2,000, and the porosity is 85%.

As can be seen from Table 7, when only the first micropores 61 are formed in the microporous sheet 312, the aerosol generated through atomization causes the lowest harshness to the throat, but the aroma, the sweetness, and the satisfaction are also low. When the number of the first micropores 61 accounts for more than 90% of the total number of micropores 6 (corresponding to a distribution solution G), factors that affect sensory attributes, such as harshness, aroma, sweetness, and satisfaction, are all moderate. Therefore, this may be more beneficial to inhalation experience of most users.

In addition, the pore size of the first aerosol outlet 611 and the pore size of the second aerosol outlet may also affect the sensory attributes. As can be seen from Table 7, when the pore size of the first aerosol outlet 611 is decreased, the harshness is also be reduced. When the pore size of the second aerosol outlet is increased, both the aroma and the sweetness are obviously improved.

Therefore, the distribution manner, the distribution area, and the pore size of the first micropores 61 and the second micropores 62 may be specifically adjusted according to different requirements. This is not specifically limited in embodiments of the present disclosure.

As shown in FIG. 10, the side, facing the liquid inlet channel 22, of the central area of the microporous sheet 312 protrudes to form a protrusion 7, and the micropores 6 are formed in the protrusion 7.

In embodiments of the present disclosure, the diameter of the protrusion 7 is between 2 mm and 8 mm, and a spacing distance between the vertex of the protrusion 7 and the plane of the microporous sheet 312 is between 1 mm and 6 mm.

For example, the diameter of the protrusion 7 may be 2 mm, 3 mm, 4 mm, 5 mm, 6 mm, 7 mm, or 8 mm; and the spacing distance between the vertex of the protrusion 7 and the plane of the microporous sheet 312 may be 1 mm, 2 mm, 3 mm, 4 mm, 5 mm, or 6 mm.

In embodiments of the present disclosure, the protrusion 7 is formed through a stamping process. The stamping process is easy to operate and has stable quality.

Of course, in some other embodiments, the protrusion 7 may alternatively be formed through any other suitable process.

As shown in FIG. 2 to FIG. 6, the atomization core 3 further includes a liquid guiding member 32. The liquid guiding member 32 is located in the liquid inlet channel 22, abuts against the protrusion 7 of the microporous sheet 312, and is used for guiding the aerosol generating substrate to the ultrasonic atomization chip assembly 31.

For example, the liquid guiding member 32 includes, but is not limited to, porous cotton, a porous ceramic, a porous plastic, or porous glass.

The liquid guiding member 32 can adsorb the aerosol generating substrate onto the top surface of the liquid guiding member, to form a thin liquid surface that is in contact with the ultrasonic atomization chip assembly 31, thereby reducing impact on a vibration effect of the ultrasonic atomization chip assembly 31 caused by an excessive liquid amount of aerosol generating substrate.

The protrusion 7 abuts against the liquid guiding member 32, to enable the micropores 6 in the microporous sheet 312 to be in contact with the liquid guiding member 32 to a maximum extent. In this way, when the atomizer 10 is in operation, atomization efficiency of the ultrasonic atomization chip assembly 31 can be improved, and when the atomizer 10 is not in operation, blockage can be achieved, thereby effectively reducing volatilization of the aroma of the aerosol generating substrate, reducing a possibility of liquid leakage from the liquid storage cavity 11, and improving user experience.

For example, the liquid guiding member 32 may be formed with a plurality of liquid guiding grooves 321, and the aerosol generating substrate in the liquid inlet channel 22 can be guided to the ultrasonic atomization chip assembly 31 by capillarity action of the liquid guiding grooves 321.

In embodiments of the present disclosure, the bottom end of the air outlet channel 12 of the atomizer 10 directly covers the periphery of the aerosol outlet area 31a used for discharging the aerosol generated through atomization. In this way, the aerosol generated through atomization can be directly discharged from the housing assembly 1 through the air outlet channel 12 for user inhalation, thereby shortening a flow path of the aerosol, reducing a possibility of condensation of the aerosol, reducing loss of the aerosol, and improving user experience.

The foregoing describes the preferred implementations of the present disclosure in detail with reference to the accompanying drawings, but the present disclosure is not limited thereto. Within the scope of the technical concept of the present disclosure, various simple variations may be made to the technical solutions of the present disclosure, including any suitable combinations of the specific technical features. To avoid unnecessary repetition, all possible combinations are not described in the present disclosure. However, these simple variations and combinations should also be considered as content disclosed in the present disclosure, and all fall within the scope of protection of the present disclosure.

## Claims

1. An atomizer, comprising:
a housing assembly, wherein an air outlet channel is formed in the housing assembly;
an atomization base, wherein at least a part of the atomization base is disposed in the housing assembly; and
an atomization core, disposed on the atomization base and comprising an ultrasonic atomization chip assembly, wherein the ultrasonic atomization chip assembly is used for atomizing an aerosol generating substrate, the ultrasonic atomization chip assembly has an aerosol outlet area used for discharging an aerosol, and the aerosol outlet area faces the top side of the atomizer, wherein
the bottom end of the air outlet channel covers the periphery of the aerosol outlet area, to cause the aerosol generated through atomization to be discharged from the housing assembly through the air outlet channel.

2. The atomizer of claim 1, wherein the atomization base is formed with an air inlet channel, the side wall at the bottom end of the air outlet channel is formed with at least one notch, and the air inlet channel communicates with the air outlet channel through the notch.

3. The atomizer of claim 1 or 2, wherein the air outlet channel linearly extends in a top-bottom direction of the atomizer.

4. The atomizer of any one of claims 1 to 3, wherein the length of the air outlet channel is not greater than 35 mm.

5. The atomizer of any one of claims 1 to 4, wherein the length of the air outlet channel is between 15 mm and 35 mm; and/or the equivalent diameter of the air outlet channel is between 2.5 mm and 5.0 mm.

6. The atomizer of any one of claims 1 to 5, wherein a liquid storage cavity is formed in the housing assembly, the liquid storage cavity is located on the top side of the atomization base, the air outlet channel is located in the middle area of the liquid storage cavity, and the ultrasonic atomization chip assembly is arranged perpendicular to the top-bottom direction of the atomizer.

7. The atomizer of any one of claims 1 to 6, wherein the atomization base comprises: an atomization top base, an atomization bottom base, and a first sealing member, wherein the atomization bottom base is located at the bottom side of the atomization top base, and the first sealing member is located at the top side of the atomization top base.

8. The atomizer of claim 7, wherein the bottom surface of the first sealing member is formed with a plurality of convex points;
the atomization top base is formed with a groove, and the ultrasonic atomization chip assembly is disposed in the groove; and
the first sealing member abuts against the ultrasonic atomization chip assembly through the convex points, to fix the ultrasonic atomization chip assembly in the groove.

9. The atomizer of claim 8, wherein a spacing area is formed between the bottom surface of the first sealing member and the top surface of the ultrasonic atomization chip assembly, and the convex points are located in the spacing area; and
the atomization top base is formed with at least one communication groove, the atomization bottom base is formed with at least one air inlet hole, the communication groove communicates with the air inlet hole and the spacing area, and the air inlet hole, the communication groove, and the spacing area define at least a part of the air inlet channel.

10. The atomizer of any one of claims 1 to 9, wherein the atomizer comprises: a second sealing member formed with a first through hole, an annular groove is formed in the hole wall of the first through hole, the second sealing member surrounds the ultrasonic atomization chip assembly, and the edge of the ultrasonic atomization chip assembly is embedded into the annular groove.

11. The atomizer of any one of claims 1 to 10, wherein the ultrasonic atomization chip assembly comprises a piezoelectric ceramic sheet and a microporous sheet, wherein
the microporous sheet is superposed on the bottom side of the piezoelectric ceramic sheet and is connected to the piezoelectric ceramic sheet, a second through hole is formed in the middle area of the piezoelectric ceramic sheet, and micropores are formed in an area, corresponding to the second through hole, of the microporous sheet.

12. The atomizer of claim 11, wherein
the micropores comprise first micropores, the first micropores form a first aerosol outlet on the surface on the side, facing the piezoelectric ceramic sheet, of the microporous sheet, and the pore size of the first aerosol outlet is not greater than 2.0 µm.

13. The atomizer of claim 12, wherein the pore size of the first aerosol outlet is between 1.1 µm and 2.0 µm.

14. The atomizer of claim 12 or 13, wherein the first micropores form a first liquid inlet on the surface on the side, facing away from the piezoelectric ceramic sheet, of the microporous sheet, and the pore size of the first liquid inlet is between 20 µm and 50 µm.

15. The atomizer of any one of claims 11 to 14, wherein a number of micropores is between 500 and 4,000; and/or
a spacing distance between any two adjacent micropores is between 40 µm and 100 µm.

16. The atomizer of any one of claims 11 to 15, wherein the thickness of the piezoelectric ceramic sheet is between 0.4 mm and 0.8 mm; and/or
the diameter of the piezoelectric ceramic sheet is between 12 mm and 14 mm.

17. The atomizer according to any one of claims 11 to 16, wherein the thickness of the microporous sheet is between 0.2 mm and 1.0 mm; and/or
the diameter of the microporous sheet is between 12 mm and 14 mm.

18. The atomizer of any one of claims 11 to 17, wherein the atomization base is formed with a liquid inlet channel, the side, facing the liquid inlet channel, of the central area of the microporous sheet protrudes to form a protrusion, and the micropores are formed in the protrusion.

19. The atomizer of claim 18, wherein the atomization core further comprises: a liquid guiding member located in the liquid inlet channel, abuts against the protrusion, and is used for guiding the aerosol generating substrate to the ultrasonic atomization chip assembly.

20. The atomizer of any one of claims 11 to 19, wherein
a material of the piezoelectric ceramic sheet is one of lead zirconate titanate, barium carbonate, and potassium sodium niobate; and/or
a material of the microporous sheet is one of stainless steel, nickel-cobalt alloy, titanium alloy, copper alloy, and palladium-nickel alloy.

21. The atomizer of any one of claims 12 to 20, wherein the micropores further comprise second micropores, the second micropores form a second aerosol outlet on the surface on the side, facing the piezoelectric ceramic sheet, of the microporous sheet, and the pore size of the second aerosol outlet is between 5 µm and 20 µm.

22. The atomizer of claim 21, wherein a ratio of a number of first micropores to a number of second micropores ranges from 7: 1 to 10: 1.

23. An ultrasonic atomization chip assembly used in the atomizer of any one of claims 1 to 22, wherein the ultrasonic atomization chip assembly comprises:
a piezoelectric ceramic sheet, wherein a second through hole is formed in the middle area of the piezoelectric ceramic sheet; and
a microporous sheet, coaxially superposed on the first axial side of the piezoelectric ceramic sheet and connected to the piezoelectric ceramic sheet, wherein micropores are formed in an area, corresponding to the second through hole, of the microporous sheet, wherein
the micropores comprise first micropores, the first micropores form a first aerosol outlet on the surface on the side, facing the piezoelectric ceramic sheet, of the microporous sheet, and the pore size of the first aerosol outlet is not greater than 2.0 µm.

24. The ultrasonic atomization chip assembly of claim 23, wherein the pore size of the first aerosol outlet is between 1.1 µm and 2.0 µm.

25. The ultrasonic atomization chip assembly of claim 23 or 24, wherein the first micropores form a first liquid inlet on the surface on the side, facing away from the piezoelectric ceramic sheet, of the microporous sheet, and the pore size of the first liquid inlet is between 20 µm and 50 µm.

26. The ultrasonic atomization chip assembly of any one of claims 23 to 25, wherein a number of micropores is between 500 and 4,000; and/or
a spacing distance between any two adjacent micropores is between 40 µm and 100 µm.

27. The ultrasonic atomization chip assembly of any one of claims 23 to 25, wherein the thickness of the piezoelectric ceramic sheet is between 0.4 mm and 0.8 mm; and/or
the diameter of the piezoelectric ceramic sheet is between 12 mm and 14 mm.

28. The ultrasonic atomization chip assembly of any one of claims 23 to 27, wherein the thickness of the microporous sheet is between 0.2 mm and 1.0 mm; and/or
the diameter of the microporous sheet is between 12 mm and 14 mm.

29. The ultrasonic atomization chip assembly of any one of claims 23 to 28, wherein the side, facing away from the piezoelectric ceramic sheet, of the central area of the microporous sheet protrudes to form a protrusion, and the micropores are located in the protrusion.

30. The ultrasonic atomization chip assembly of any one of claims 23 to 29, wherein a material of the piezoelectric ceramic sheet is one of lead zirconate titanate, barium carbonate, and potassium sodium niobate; and/or
a material of the microporous sheet is one of stainless steel, nickel-cobalt alloy, titanium alloy, copper alloy, and palladium-nickel alloy.

31. The ultrasonic atomization chip assembly of any one of claims 23 to 30, wherein the micropores further comprise second micropores, the second micropores form a second aerosol outlet on the surface on the side, facing the piezoelectric ceramic sheet, of the microporous sheet, and the pore size of the second aerosol outlet is between 5 µm and 20 µm.

32. The ultrasonic atomization chip assembly of claim 31, wherein a ratio of a number of first micropores to a number of second micropores ranges from 7: 1 to 10: 1.

33. An aerosol generating device, comprising:
a power supply component; and
the atomizer of any one of claims 1 to 22, wherein
the power supply component is electrically connected to the atomizer and is used for supplying power to the atomizer.
